(19) Europäisches Patentamt
European Patent Office
Office européen des brevets

(11) Veröffentlichungsnummer: **0 195 300**
**A2**

(12) # EUROPÄISCHE PATENTANMELDUNG

(21) Anmeldenummer: **86102763.9**

(22) Anmeldetag: **03.03.86**

(51) Int. Cl.[4]: **C07D 261/02**, A01N 43/80, C07C 15/44, C07C 83/02

(30) Priorität: **16.03.85 DE 3509547**

(43) Veröffentlichungstag der Anmeldung: **24.09.86 Patentblatt 86/39**

(84) Benannte Vertragsstaaten: **AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Ingendoh, Axel, Dr.**
**Unterste Dillenberg 18**
**D-5620 Velbert 15(DE)**
Erfinder: **Scheinert, Wolfgang, Dr.**
**Emil-Nolde-Strasse 41**
**D-5090 Leverkusen 1(DE)**
Erfinder: **Becker, Benedikt, Dr.**
**Metzkausener Strasse 14**
**D-4020 Mettmann(DE)**
Erfinder: **Halcour, Kurt, Dr.**
**Gellertstrasse 14**
**D-5090 Leverkusen 1(DE)**
Erfinder: **Stendel, Wilhelm, Dr.**
**In den Birken 55**
**D-5600 Wuppertal 1(DE)**

(54) **Isoxazolidine, Verfahren zu ihrer Herstellung und ihre Verwendung als Schädlingsbekämpfungsmittel.**

(57) Die vorliegende Erfindung betrifft neue Isoxazolidine, der Formel I

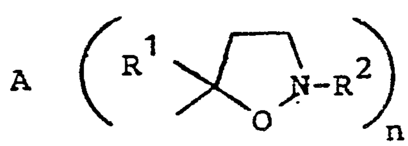

I

in welcher

n für 2 oder 3 steht,

$R^1$ für H oder Alkyl steht,

$R^2$ für gegebenenfalls substituiertes Alkyl, Cycloalkyl, Alkenyl, Alkinyl, Aryl sowie für gesättigte oder ungesättigte heterocyclische Reste die gegebenenfalls substituiet sind, steht,

A für zwei oder dreiwertige Aryl-oder Heteroarylreste steht, die gegebenenfalls durch Substituenten aus der Reihe Alkyl, Aryl, Aralkyl, Alkoxy, Alkylthio, Halogen, CN, OH, Halogenalkyl, Halogenalkoxy, Halogenalkylthio, Alkoxyalkyl, gegebenenfalls halogensubstituiertes Methylendioxy oder Ethylendioxy substituiert sind.

Die neuen Isoxazolidine der Formel I können in Form ihrer Enantiomere, Diastereomere sowie als Salze mit anorganischen oder organischen Säuren vorliegen.

Sie eignen sich als Schädlingsbekämpfungsmittel.

EP 0 195 300 A2

Isoxazolidine, Verfahren zu ihrer Herstellung und ihre Verwendung als Schädlingsbekämpfungsmittel

Die vorliegende Erfindung betrifft neue Isoxazolidine, Verfahren zu ihrer Herstellung, ihre Verwendung als Schädlingsbekämpfungsmittel und neue Zwischenprodukte zu ihrer Herstellung sowie Verfahren zu deren Herstellung.

Isoxazolidine sind bereits bekannt geworden (J. Org. Chem. 44, 45 (1979) S. 835-839). Es ist jedoch nichts über biologische Eigenschaften und insbesondere über ihre Eignung als Schädingsbekämpfungsmittel bekannt geworden.

Es wurden die neuen Isoxazolidine der Formel I gefunden

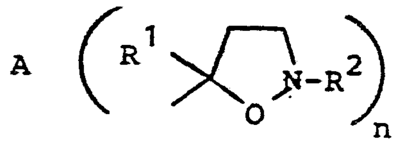

I

in welcher

n für 2 oder 3 steht,

$R^1$ für H oder Alkyl steht,

$R^2$ für gegebenenfalls substituiertes Alkyl, Cycloalkyl, Alkenyl, Alkinyl, Aryl sowie für gesättigte oder ungesättigte heterocyclische Reste die gegebenenfalls substituiert sind, steht,

A für zwei-oder dreiwertige Aryl-oder Heteroaryl-reste steht, die gegebenenfalls durch Substituenten aus der Reihe Alkyl, Cycloalkyl, Aryl, Aralkyl, Alkoxy, Alkylthio, Aryloxy, Arylthio, Halogen, CN, OH, Halogenalkyl, Halogenalkoxy, Halogenalkylthio, Alkoxyalkyl, gegebenenfalls halogensubstituiertes Methylendioxy oder Ethylendioxy substituiert sind.

Die neuen Isoxazolidine der Formel I können in Form ihrer Enantiomere, Diastereomere sowie als Salze mit anorganischen oder organischen Säuren vorliegen.

Es wurde ein Verfahren zur Herstellung der neuen Isoxazolidine der Formel I gefunden

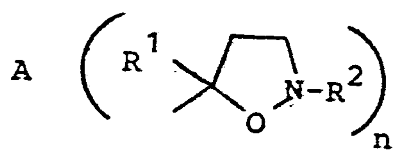

I

in welcher

n für 2 oder 3 steht,

$R^1$ für H oder Alkyl steht,

$R^2$ für gegebenenfalls substituiertes Alkyl, Cycloalkyl, Alkenyl, Alkinyl, Aryl sowie für gesättigte oder ungesättigte heterocyclisch Reste die gegebenfalls substituiert sind, steht,

A für zwei-oder dreiwertige Aryl-oder Heteroarylreste steht, die gegebenenfalls noch weitere Substituenten aus der Reihe Alkyl, Cycloalkyl, Aryl, Aralkyl, Alkoxy, Alkylthio, Aryloxy, Arylthio, Halogen, CN, OH, Halogenalkyl, Halogenalkoxy, Halogenalkylthio, Alkoxyalkyl, gegebenenfalls halogensubstituierte Methylendioxy oder Ethylendioxy substituiert sind,

das durch gekennzeichnet ist, daß man Hydroxylamine der Formel II

$R^2$-NH-OH II

in welcher

$R^2$ die oben angegebene Bedeutung hat

mit Formaldehyd und Alkenen der Formel III

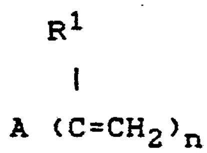

III

in welcher

A, $R^1$, n die oben angegebene Bedeutung haben

umsetzt.

Die Hydroxylamine der Formel II sind teilweise neu. Es wurden die neuen alkylsubstituierten Cycloalkylhydroxylamine der Formel IV gefunden

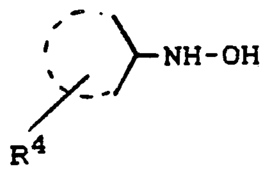

IV

in welcher

$R^4$ für Alkyl steht.

Die alkylsubstituierten Cycloalkylhydroxylamine der Formel IV werden erhalten, indem man alkylsubstituierte Cycloalkanonoxime der Formel V

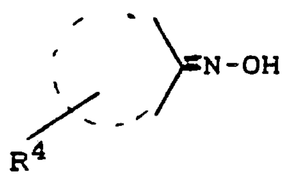

V

in welcher

$R^4$ für Alkyl steht,

reduziert.

Alkylsubstituierte Cycloalkanonoxime der Formel V sind bekannt oder lassen sich analog zu bekannten Verfahren herstellen (Tetrahedron, 1967, 23(5)2421-9).

Die Alkene der Formel III sind teilweise bekannt oder lassen sich analog zu bekannten Verfahren herstellen. 3,5-Diisopropenylkumol ist neu.

Solche Verfahren zur Herstellung der Alkene der allgemeinen Formel III sind z.B.

a) Eliminierung von HX aus Verbindungen der allgemeinen Formel VI

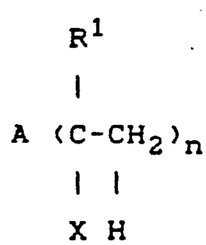

VI

in welcher

A, $R^1$ und n die in der Formel I angegebene Bedeutung haben und

X für Wasserstoff, Halogen, Hyroxyl, $OSO_2$-$R^3$, OCO-$R^3$ ($R^3$ = $C_1$-$C_4$-Alkyl) steht, und

b) Eliminierung von HX aus Verbindungen der allgemeinen Formel VII

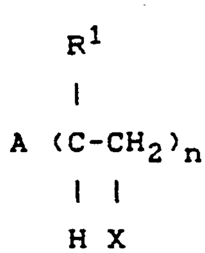

VII

in welcher

A, $R^1$, n und X die in der Formel VI angegebene Bedeutung haben,

c) Olefinierung (nach Wittig) von Verbindungen der allgemeinen Formel VIII

$$\begin{array}{c} R^1 \\ | \\ A\ (C=O)_n \end{array} \qquad VIII$$

in welcher

A, $R^1$ und n die in der Formel I angegebene Bedeutung haben,

mit Yliden der allgemeinen Formel IX

$$W_3\ P = CH_2 \qquad IX$$

in welcher

W für z.B. Phenyl steht.

Isoxazolidine der Formel I eignen sich zur Bekämpfung von Schädlingen, insbesondere zur Bekämpfung schädlicher Milben und Insekten. Die Verbindungen besitzen auch hervorragende fungizide Wirkungen. Da von den bekannten Isoxazolidinen keinerlei biologische Wirksamkeit bekannt war, war es überraschend, daß die erfingungsgemäßen Verbindungen eine so ausgezeichnete Wirkung als Schädlingsbekämpfungsmittel und insbesondere als Insektizide, Akarizide und Fungizide besitzen.

Von den erfindungsgemäßen Isoxazolidinen der Formel I sind bevorzugt diejenigen bei denen

A für gegebenenfalls durch $C_{1-4}$-Alkyl substituiertes Aryl insbesondere Phenyl oder Naphthyl steht,

n für 2 oder 3 steht,

$R^1$ für Wasserstoff oder $C_{1-4}$-Alkyl steht,

$R^2$ für $C_{1-10}$-Alkyl, $C_{3-6}$-Cycloalkyl, $C_{2-4}$-Alkenyl, $C_{2-4}$-Alkinyl, Phenyl, Naphthyl steht, für $C_{1-6}$ -Alkyl steht das durch Halogen, OH, CN, Amino, $C_{1-4}$-Alkylamino, $C_{1-4}$-Dialkylamino, $C_{1-4}$-Alkoxy, $C_{3-6}$-Cycloalkyl, gegebenenfalls substituiertes Phenyl, Naphthyl, Phenoxy, Phenylthio, Oxycarbonyl-$C_{1-4}$-alkyl, (OCO-$C_{1-4}$ -Alkyl), substituiert ist, für $C_{3-6}$-Cycloalkyl steht, das durch $C_{1-6}$-Alkyl, Halogen, CN, substituiert ist, für Phenyl steht das durch Halogen, $NO_2$, CN, OH, Amino, $C_{1-4}$-Alkylamino, Di-$C_{1-4}$-alkylamino, Carbonyl-($C_{1-4}$-alkoxy) (-COO$C_{1-4}$-Alkyl) substituiert ist, für gegebenenfalls substituiertes Phenoxy, Naphthoxy, Biphenyloxy steht.

Ganz besonders bevorzugt sind diejenigen Isoxazolidine der Formel I bei denen

A für Phenyl steht das gegebenenfalls durch $C_{1-4}$-Alky insbesondere Isopropyl substituiert ist,

n für 2 oder 3 steht,

$R^1$ für Wasserstoff oder Methyl steht,

$R^2$ für $C_{1-6}$-Alkyl insbesondere, Methyl, Ethyl, Butyl, t-Butyl, Pentyl, Pinakolyl, Hexyl (und deren Isomere) steht, die gegebenefalls durch Cyclopropyl substituiert sein können, sowie für Cyclohexyl steht, das gegebenenfalls durch $C_{1-6}$ -Alkyl substituiert sein kann, steht.

Ganz besonders bevorzugt sind Verbindungen der Formel I in welcher $R^2$ für verzweigtes Alkyl mit mindestens 4 C-Atomen, die gegebenenfalls durch Cyclopropyl substituiert sein können, sowie für Cyclohexyl steht, das gegebenenfalls durch $C_{4-6}$ -verzweigtes Alkyl substituiert ist.

Im einzelnen seien folgende Verbindungen genannt:

| n | A | $R^1$ | $R^2$ |
|---|---|---|---|
| 2(in 2,5 Stellung) | Pyridyl | H | Pinakolyl |
| 2(in 1,7 Stellung) | Naphthyl | H | 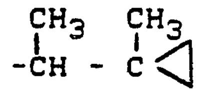 |
| 2(in 2,4 Stellung) | Thienyl | H | Isopropyl |
| 2(in 5,7 Stellung) | Chinolyl | $CH_3$ | 2-t-Butyl-hexyl |
| 2(in 1,3 Stellung) | 5-Brom-phenyl | $CH_3$ | Pinakolyl |

Die Verbindungen der Formel I können in Form ihrer Diasteromeren und Enantiomeren aufgrund der mit A und B in der folgenden Formel bezeichneten asymmetrischen C-Atome vorliegen:

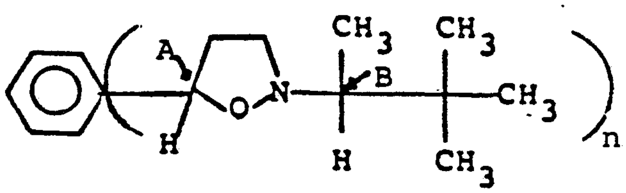

Die Verbindungen der Formel I können in Form ihrer Salze vorliegen.

Bevorzugt seien folgende Säuren genannt, die mit den Isoxazolidinen der Formel I Salze bilden können:

HCl, $H_2SO_4$, $HSO_4^-$ $H_3PO_4$, $HPO_4^{2-}$, $HClO_4$, HBr, HJ, HF, $HNO_3$, $H_2CO_3$, $HCO_3^-$, Essigsäure, Oxalsäure, Malonsäure, Bernsteinsäure, Äpfelsäure, Weinsäure, Maleinsäure, Fumarsäure, Methansulfonsäure, Benzoesäure, substituierte Benzoesäuren, Ameisensäure, Chloressigsäure, Toulsulfonsäure, Benzolsulfonsäure, Trichloressigsäure, Fluoressigsäure, Phthalsäure, Naphthalinsulfonsäure, Nikotinsäure.

Das Verfahren zur Herstellung der Isoxazolidine der Formel I kann so durchgeführt werden, daß die Hydroxylamine der Formel II, Formaldehyd und die Alkene der Formel III zusammengegeben werden und zur Reaktion gebracht werden. Es läßt sich durch folgendes Formelschema darstellen:

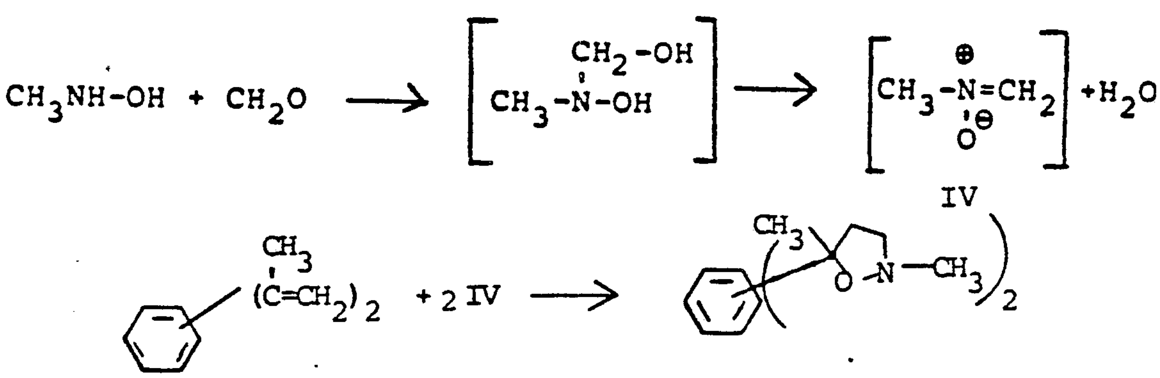

IV

Die zur Herstellung der Isoxazolidine der Formel I eingesetzten Hydroxylamine der Formel I sind an sich bekannt oder lassen sich nach an sich bekannten Verfahren herstellen. In Formel II hat $R^2$ bevorzugt die für die Verbindungen der Formel I angegebenen bevorzugten Bedeutungen.

Besonders seien die folgenden Hydroxylamine der Formel II genannt:

$CH_3NHOH$, 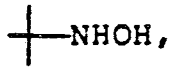, Phenyl-$CH_2$-NHOH, 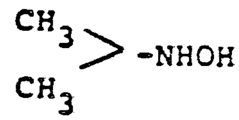,

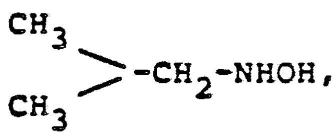, 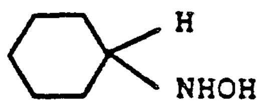, 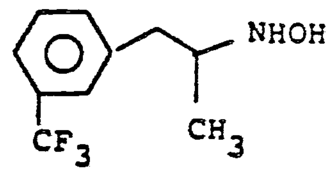

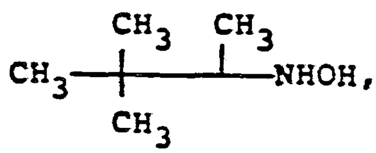, 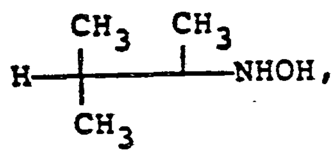, 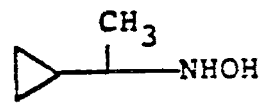

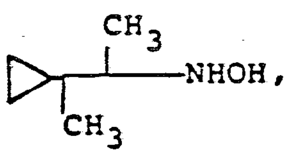, 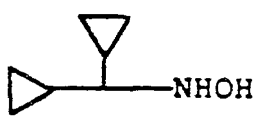, 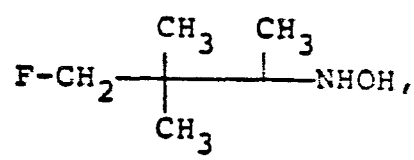,

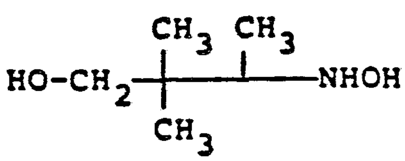, 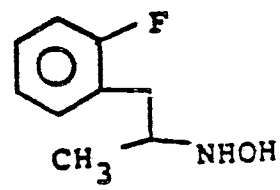

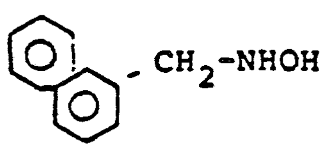, 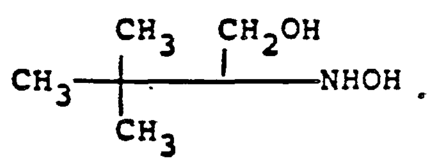.

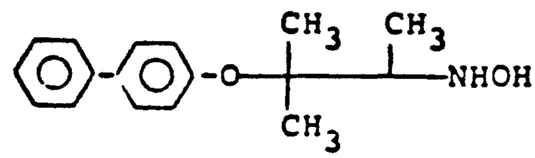 ,

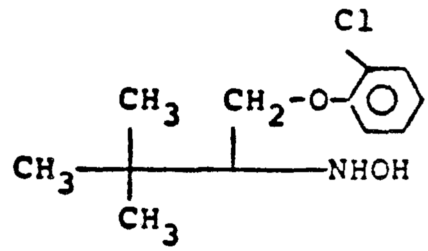 ,

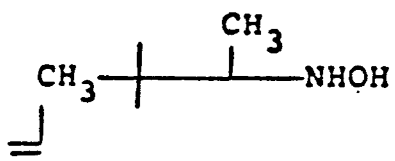 ,

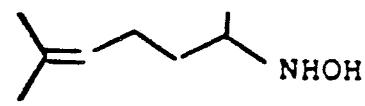 ,

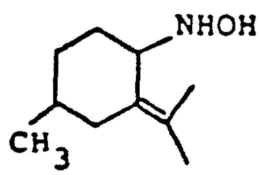

2-tert.-Butyl-cyclohexylhydroxylamin ist ebenfalls besonders bevorzugt. Er ist neu, seine Herstellung wird weiter unten beschrieben.

Zur Herstellung der Isoxazolidine der Formel I wird Formaldehyd in Form von Formalin, Paraformaldehyd oder von Formaldehyd abspaltenden Verbindungen wie Trimethylenoxid, eingesetzt.

Zur Herstellung der Isoxazolidine der Formel I werden Alkene der Formel III eingesetzt in der die Reste A und R' die bei den Verbindungen der Formel I genannten be vorzugten und besonders bevorzugten Bedeutungen haben. Im einzelnen seien die in den Beispielen genannten Alkene der Formel III genannt.

Das Verfahren zur Herstellung der erfindungsgemäßen Verbindungen der Formel I wird bei Temperaturen von 0-100°C bevorzugt bei 100-140°C durchgeführt.

Pro olefinische Doppelbindung der Alkene der Formel III wird 1-2 bevorzugt 1-1,5 Äquivalent Hydroxylamin der Formel II und 1-2,5 bevorzugt 1-1,5 Äquivalente Formaldehyd eingesetzt.

Es wird normalerweise bei Normaldruck gearbeitet. Es wird bevorzugt in Gegenwart von Verdünnungsmitteln gearbeitet. Geeignete Verdünnungsmittel sind gegebenenfalls substituierte Kohlenwasserstoffe wie Toluol, Xylol, Benzol, Chloroform, Alkohole wie Ethanol, Isopropanol, Ether, wie Dialkylether, Ester wie Essigester, ferner THF, Acetonitril, DMF, DMSO.

Es ist auch möglich die Hydroxylamine der Formel II mit Formaldehyd zu versetzen und anschließend die Alkene der Formel III zum Reaktionsgemisch zuzugeben.

Est ist auch möglich.nach einer besonders bevorzugten Verfahrensweise die Hydroxylamine der Formel II in Form ihrer Salze mit anorganischen Säuren, insbesondere HCl, sowie Formaldehyd, bevorzugt in Form seiner wäßrigen Formalinlösung, zur Reaktion zu bringen und anschließend das Alken der Formel III der Reaktionslösung zu geben.

Es wird bei Temperaturen von 20-150°C unter Normaldruck gearbeitet.

Formaldehyd kann auch im Überschuß eingesetzt werden (ca. 1-5fach pro Doppelbindung)

Als geeignete Verdünnungsmittel seien dabei inerte organische Verdünnungsmittel wie Alkohole wie Methanol, Ethanol, Isopropanol, Kohlenwasserstoffe wie Toluol, DMF, DMSO, hochsiedende Ether genannt.

Als starke anorganische Basen seine Alkali-und Erdalkalihydroxide wie Natronlauge oder Kalilauge, Alkali-und Erdalkalicarbonate und Hydrogencarbonate genannt.

Als geeignete organische Basen seien genannt:

Trimethylamin, Triethylamin, N-Ethyldiisopropylamin, N,N-Dimethylanilin, N,N-Diethylanilin, N,N,N',N'-Tetra-methylethylendiamin, N,N,N',N'-Tetraethylethylendiamin, Lutidin, Picolin, Pyridin.

Die Neutralisation erfolgt etwa bei 20°C zu einem pH-Wert von 6-8.

Anschließend wird zum Reaktiongemisch das Alken der Formel III sowie ein "Wasserschleppmittel" zugegeben.

Als Wasserschleppmittel seien genannt:

Aromatische Kohlenwasserstoffe wie insbesondere Benzol, Toluol, Xylol, sowie halogenierte Kohlenwasserstoffe wie Chloroform, Tetrachlorkohlenstoff.

Danach wird das Reaktionsgemisch auf 110-140°C erhitzt. Alkohol und Wasser werden mit Hilfe des Wasserschleppmittels azeotrop abdestilliert.

Nachdem der Alkohol abdestilliert ist und das Wasser mit dem Wasserschleppmittel aus dem Reaktionsgemisch entfernt worden ist läßt man bei 110-140°C nachreagieren und arbeitet dann in üblicher Weise auf.

Wie bereits erwähnt sind die Hydroxylamine der Formel IV neu. Das Verfahren zu ihrer Herstellung läßt sich für den Fall, daß 4-Methylcyclohexanonoxim und Na $BH_3CN$ verwendet werden, durch folgendes Formelschema darstellen:

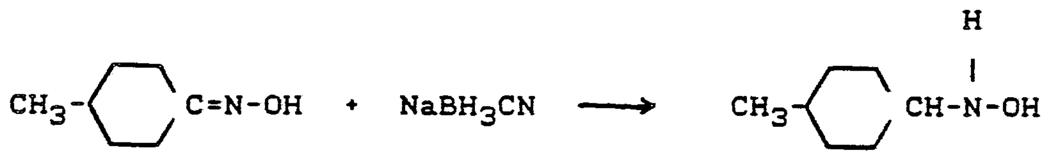

Besonders bevorzugt seien die neuen alkylsubstituierten Cycloalkylhydroxylamine der Formel IV genannt, in welcher der Cycloalkylring 4 -6 C-Atome enthält. Ganz besonders bevorzugt seien als Cycloalkylringe Cyclobutan, Cyclopentan und Cyclohexan genannt. Insbesondere sei Cyclohexan genannt.

Als Substituenten der Cycloalkylringe seien Alkylreste mit 1 -7 C-Atomen insbesondere mit 3 -5 C-Atomen genannt. Besonders bevorzugt seien als Alkylsubstituenten verzweigte Alkylreste mit 3 -5 C-Atomen wie z.B. t-Butyl genannt.

Besonders zu erwähnen ist t-Butyl-cyclohexanon-hydroxylamin.

Die zur Herstellung der Hydroxylamine der Formel IV eingesetzten Cycloalkanonoxime der Formel V sind bekannt oder lassen sich analog zu bekannten Verfahren herstellen (Tetrahedron 1967, 23(5) S. 2421-9.

Bevorzugt werden die Cycloalkanonoxime eingesetzt die zu den obengenannten bevorzugten Cycloalkanonhydroxylaminen führen.

Als Reduktionsmittel besonders geeignet sind Alkalicyanoborhydride wie natriumcyanoborhydrid. Die Reduktion läßt sich jedoch auch mit anderen geeigneten Reduktionsmitteln durchführen.

Die Reduktion der Cycloalkanonoxime mit Natriumcyanoborhydrid wird bei -20°C bis +100°C, bevorzugt bei etwa 20°C, durchgeführt.

Es wird bei Normaldruck gearbeitet.

Die Ausgangskomponenten werden etwa in äquimolarem Verhältnis eingesetzt.

Es wird in Verdünnungsmitteln gearbeitet. Als solche seien genannt: Alkohole wie Methanol, Ethanol, Isopropanol, Ethylenglycol. Es kann auch in wäßrigen Alkoholen gearbeitet werden.

Es wird bei einem pH-Wert der Reaktionslösung von etwa 1-4 gearbeitet. Der pH-Wert kann durch eine geeichte Glaselektrode bestimmt werden. Ferner ist die Zugabe eines Farbindikators wie Bromkresolgrün oder Methylorange zur Einstellung des pH-Wertes geeignet.

Die Aufarbeitung kann in an sich bekannter Weise durch Ansäuern des Ansatzes mit konzentrierter Säure wie Salzsäure zur Zersetzung des Cyanoborhydrids und anschließendem Alkalisieren, gefolgt von einer Extraktion des N-Alkylhydroxylamins mit organischen Lösungsmitteln erfolgen (vgl. R. Borch J.Am.Chem. Soc. 93, 2897 (1971)).

Besonders vorteilhaft ist es jedoch, nach Beendigung der Borhydridreduktion und Zersetzung des Borhydrids mit konzentrierter Säure wie Salzsäure den Ansatz im Wasserstrahlvakuum einzuengen, den Rückstand mit Chloroform oder Dichlormethan oder Alkoholen wie Ethanol, Isopropanol oder Methanol zu extrahieren. Die organische Lösung des N-Alkylhydroxylamin-Hydrochlorids wird mit Natriumsulfat oder anderen geeigneten Trocknungsmitteln getrocknet und am Rotationsverdampfer eingeengt.

Die Verbindungen der Formel IV können auch in an sich bekannter Weise durch Reduktion der entsprechenden Nitroverbindungen mit Wasserstoff unter Katalyse von z.B. Palladium auf Kohle (vgl. US-P 3 173 953) oder durch Reduktion mit Zinkstaub in Eisessig, mit amalgamiertem Aluminium, mit Zinn-(II)-chlorid (vgl. Houben Weyl Methoden der org. Chemie Band 10/1 S. 1153) hergestellt werden.

Die Herstellung der als Zwischenprodukte eingesetzten besonders bevorzugten Alkene der allgemeinen Formel III in welcher

A für Phenyl,

$R^1$ für Wasserstoff oder $C_1$-$C_3$-Alkyl steht und

n für 2 oder 3 steht

kann z.B. erfolgen durch katalytische Dehydrierung von Verbindungen der allgemeinen Formeln VI oder VII (s. oben) in denen X für Wasserstoff steht.

Die katalytische Umsetzung kann auf an sich bekannte Weise erfolgen, z.B. in der Gasphase bei hohen Temperaturen (z.B. 400-700°C = 673-973 K) bei Normaldruck oder erhöhtem Druck unter Ausschluß oder in Gegenwart von Sauerstoff, sauerstoffhaltigen Verbindungen wie z.B. Carbonylsulfid oder anderen Verbindungen, die wie z.B. Schwefel durch Bindung von Wasserstoff die Dehydrierung der in die Reaktion eingesetzten Verbindungen bewirken können. Dabei kann auch von der an sich bekannten und technisch durchgeführten Herabsetzung des Partialdruckes der eingesetzten organischen Verbindung durch Wasserdampfzusatz Gebrauch gemacht werden, wodurch die Katalysatoraktivität gegebenenfalls länger aufrecht erhalten werden kann.

Als Katalysatoren können z.B. für die katalytische Dehydrierung ohne Sauerstoff, sauerstoffhaltige Verbindungen oder Verbindungen, die durch Bindung von Wasserstoff die Dehydrierung bewirken können, unter den genannten Druck-und Temperaturbedingungen handelsübliche Kontakte z.B. auf $Fe_2O_3$-oder $Cr_2O_3$-Basis verwendet werden, die außerdem weitere Komponenten wie z.B. Alkalicarbonate sowie andere Metalloxide enthalten können.

Analog können zur Dehydrierung der eingesetzten organischen Verbindung in der Gasphase bei hohen Temperaturen (z.B. 300-600°C = 573-873 K) bei Normaldruck oder erhöhtem Druck in Gegenwart von Sauerstoff, sauerstoffhaltigen Verbindungen oder Verbindungen, die durch Bindung von Wasserstoff dehydrierend wirken können, z.B. phosphathaltige Katalysatoren verwendet werden, die für diesen Reaktionstyp besonders geeignet sind.

Die Reaktion kann bei diesen Dehydrierungen in den technisch üblichen Reaktoren durchgeführt werden, die den Katalysator in Form eines Festbettes enthalten, z.B. in durch Rauchgas oder durch eine Salzschmelze beheizten Rohrreaktoren, die eine mehr oder weniger isotherme Temperaturführung erlauben oder alternativ z.B. in adiabatisch arbeitenden, rauchgasbeheizten Hordenreaktoren. Zur Erzielung einer besonders wirtschaftlichen Betriebsweise kann das anfallende flüssige Produktgemisch nach Abtrennung der angestrebten Zielverbindungen zusammen mit dem Einsatzstoff erneut dem Reaktor zugeführt werden.

Die Wirkstoffe eignen sich bei guter Pflanzenverträglichkeit und günstiger Warmblütertoxizität zur Bekämpfung von tierischen Schädlingen, isbesondere Insekten, Spinnentieren und Nematoden, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonela, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe eignen sich außerdem zur Bekämpfung pilzlicher Pflanzenkrankheiten wie z.B. Pyricularia oryzae in Reis.

Die Wirkstoffe können in die üblichen Formulierungen überführt werden, wie Lösungen, Emulsionen, Suspensionen, Pulver, Schäume, Pasten, Granulate, Aerosole, Wirkstoffimprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmneben-Formulierungen.

Diese Formulierungen werden in bekannter Weise hergestellt, z. B. durch Vermischen der Wirkstoffe mit Streckmitteln, also flüssigen Lösungsmitteln, unster Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in

Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkalsulfonate, Alkylsulfate, Arylsulfonate sowie Ei weißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetable Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennänhrstoffe wie Salze von Eisen, Mangan, Bor, Kupfer, Kobalt, Molybdän und Zink verwendet werden.

Die Formulierungen enthalten im allgemeinen zwischen O,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene-und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto-und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens - (Sprayen), Aufgießens (pour-on and spot-on) und des Einpuderns sowie durch parenterale Anwendung in Form beispielsweise der Injektion.

Beispiel A

Test mit Lucilia cuprina resistent-Larven

Emulgator: 35 Gewichtsteile Ethylenglykolmonomethylether

35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 1, 3, 8, 9.

Beispiel B

Test mit Boophilus microplus resistent

Lösungsmittel: 35 Gewichtsteile Ethylenglykolmonomethylether

35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik: 1, 3, 8, 9.

Beispiel C

Laphygma-Test

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1, 3, 8, 9.

Beispiel D

Tetranychus-Test (resistent)

Lösungsmittel: 7 Gewichtsteile Dimethylformamid.

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 1, 3, 8, 9.

Herstellungsbeispiele

Allgemeine Verfahrensvorschrift:

Eine Lösung von 0,1 Mol des Bis-oder Tris-alkens der Formel II in 250 ml Toluol wird auf 100°C erhitzt und mit einer alkoholisch wäßrigen Lösung, die erhalten wird indem man 0,22 Mol N-Alkylhydroxylamin-Hydrochlorid der allgemeinen Formel III mit 0,22 Mol einer wäßrigen Formalin-Lösung (33%) versetzt, mit Kaliumhydroxid-Lösung (1 molar) in Methanol neutralisiert, vom Kaliumchlorid abfiltriert, versetzt.

Dabei destilliert zunächst Methanol ab, danach wird auf 140°C Außentemperatur erwärmt. Dabei destillieren Toluol und Wasser azeotrop ab. Es wird weitere 5 Stunden unter Rückfluß gekocht. Man läßt erkalten, filtriert feste Rückstände ab und versetzt mit 100 ml 10 % wäßriger Salzsäure. Man trennt vom Toluol ab und ethert die wäßrige Phase noch zweimal aus. Man alkalisiert die wäßrige Phase und ethert erneut 3 x aus. Die vereinigten Etherauszüge werden über Natriumsulfat getrocknet, der Ether abdestilliert und der Rückstand im Hochvakuum destilliert oder durch Säulenchromatographie gereinigt.

Nach dieser Vorschrift wurden die Verbindugnen der folgenden Tabelle erhalten:

## T a b e l l e 3

| Beispiel Nr. | Formel | Physikalische Eigenschaften Daten der $^1H$-NMR-$CDCl_3$-Spektrums δ/ppm |
|---|---|---|
| 1 | 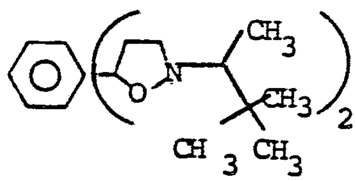<br>o:m:p=0:3:1 | 7,44-7,2 [m]<br>4,92 [n]<br>3,1-2,0 [m]<br>1,0 [1] |
| 2 | 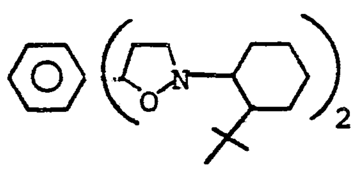<br>o:m:p=0:3:1 | 7,50-7,20 [m]<br>4,95 [t]<br>2,0-1,0 [m]<br>1,0 [s] |
| 3 | 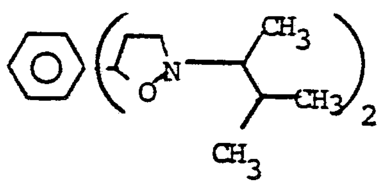<br>o:m:p=0:3:1 | 7,4-7,0 [m]<br>4,95 [b]<br>3,0-2,0 [m]<br>1,0-0,8 [3x5] |

T a b e l l e 3 (Fortsetzung)

| Beispiel Nr. | Formel | Physikalische Eigenschaften Daten der $^1H$-NMR-$CDCl_3$-Spektrums $\delta$/ppm |
|---|---|---|
| 4 | 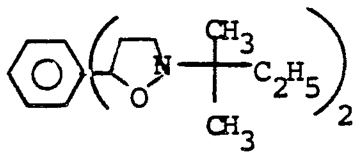<br>o:m:p=0:3:1 | 7,4-7,15 [m]<br>4,95 [b]<br>1,05 [s] |
| 5 | 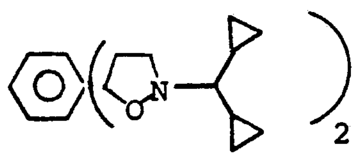<br>o:m:p=0:3:1 | 7,45-7,2 [m]<br>5,00 [t]<br>0,7-0,25 [m] |
| 6 | 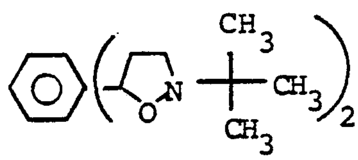<br>o:m:p=0:3:1 | 7,4-7,2 [m]<br>4,95 [t]<br>11,08 [s] |

Tabelle 3 (Fortsetzung)

| Beispiel Nr. | Formel | Physikalische Eigenschaften Daten der $^1$H-NMR-$CDCl_3$-Spektrums δ/ppm |
|---|---|---|
| 7 | 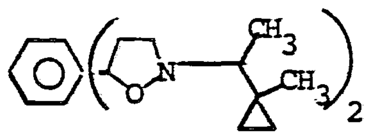<br>o:m:p=0:3:1 | 7,45-7,2 [m]<br>5,00 [t]<br>1,9-0,9 [m]<br>0,1-0,5 [m] |
| 8 | 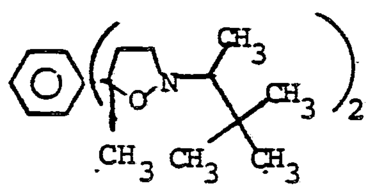<br>in 1,3-Stellung | 7,60-7,20 [m]<br>1,45 [s]<br>1,55 [s]<br>0,90 [s] |
| 9 | 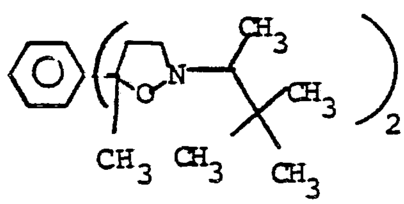<br>in 1,4 Stellung | 7;45-7,20 [m]<br>3,0-2,0 [m]<br>1,55 [s]<br>1,50 [s]<br>1,01 [s]<br>1,08 [s] |

T a b e l l e 3 (Fortsetzung)

| Beispiel Nr. | Formel | Physikalische Eigenschaften Daten der $^1H$-NMR-$CDCl_3$-Spektrums δ/ppm |
|---|---|---|
| 10 | 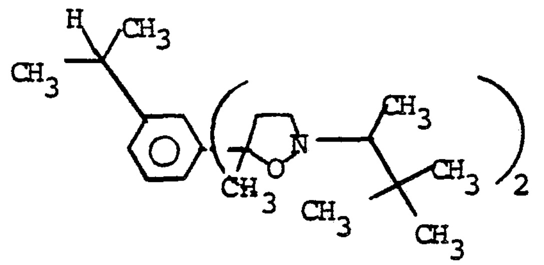<br>in 1,3-Stellung | 7,30-7,20 [m]<br>3,0-2,0 [m]<br>1,5 [s]<br>1,23 [s]<br>1,52 [s]<br>1,00 [s]<br>1,26 [s] |
| 11 | 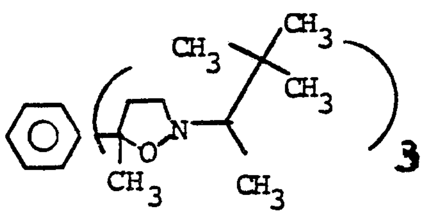<br>in 1,3,5-Stellung | 7,60-7,30 [m]<br>3,0-2,0 [m]<br>1,57 [s]<br>1,52 [s]<br>1,01 [s]<br>1,00 [s]<br>0,95 [s] |

Herstellung der Ausgangsprodukte:

Beispiel a

Folgende Vorschrift wurde zur Herstellung von Di-und Trivinylbenzolen benutzt:

Man verwendet einen elektrisch beheizten, mit einem Vorverdampfer versehenen Rohrreaktor. Das Reaktionsrohr mit 20 mm Innendurchmesser und 400 mm Länge wird über eine Strecke von ca. 350 mm elektrisch beheizt. Der Reaktor ist über eine kurze Flanschverbindung mit einem darüber angeordneten, identisch aufgebauten beheizten Rohr gleicher Abmesseungen verbunden, das als Vorverdampfer dient. Am Eingang des Vorverdampfers liegen die Einspeisungsstellen für Wasser und das zu dehydrierende Kohlenwasserstoffgemisch, die beide mit je einer Kolbendosierpumpe (Telabpumpe) der Apparatur getrennt zugeführt werden. Die Füllung des Reaktors besteht aus 100 ml eines handelsüblichen Dehydrierkontaktes (Girdler-Südchemie G 64 C mit $Fe_2O_3$ als Hauptbestandteil, Extrudat mit 2 mm Strangdurchmesser), der zur besseren Verteilung des in den Reaktor eintretenden Kohlenwaserstoff-Wasserdampfgemisches über den Rohrquerschnitt mit einer 25 ml-Schüttung aus Berl-Sattelkörpern überschichtet wird.

Der Vorverdampfer enthält eine Füllung von 125 ml Berl-Sattelkörpern. Zur Halterung der Füllungen von Reaktor und Vorverdampfer dienen Siebeinbauten, die im Boden des jeweiligen Apparates angebracht sind.

Die Elektrobeheizungen von Vorverdampfer und Reaktor werden so eingestellt, daß das Kohlenwasserstoff-Wasserdampfgemisch mit einer Temperatur von ca. 500°C (= 773 K) in den Reaktor eintritt und im Katalysatorbett ein Temperaturmaximum von ca. 630°C (= 903 K) gemessen wird. Die aus dem Reaktor austretenden Bestandteile des Reaktionsgemisches werden in einem mit Wasserkühlmantel versehenen Rundkolben aufgefangen und kondensiert. Der Rundkolben ist zur Produktentnahme mit einem Bodenablaß versehen. Zur möglichst vollständigen Gewinnung evtl. nicht kondensierter Bestandteile schaltet man in den Abgasweg hinter den Rundkolben einen Rückflußkühler.

Zur Durchführung des Dehydrierversuches werden zunächst der Vorverdampfer und der Reaktor aufgeheizt, so daß im Bereich der Reaktormitte eine Tempertur von ca. 630°C (= 903 K) und im Vorverdampfer eine Temperatur von ca. 500°C (= 773 K) gemessen werden. Nach Einstellen eines Wasserstromes von ca. 180 ml/h mit der Wasserdampf-Dosierpumpe werden die Heizleistungen von Vorderdampfer und Reaktor korrigiert, so daß sich wieder die oben angegebenen Temperaturwerte ergeben. Anschließend wird mit der Kohlenwasserstoff-Dosierpumpe ein

Kohlenwasserstoffstrom von ca. 30 ml/h eingestellt. Das im Abscheider niedergeschlagene Kondensat wird in Abständen von ca. 2 h abgelassen, die Kohlenwasserstoffphase abgetrennt und nach Zugabe von ca. 0,5 Gew.-% Tertiärbutylbrenzcatechin als Polymerisationsinhibitor der Aufarbeitung (s.u.) zugeführt.

Mit Diethylbenzol als Hauptbestandteil des Kohlenwasserstoff-Einsatzgemisches (Versuch 1) wurden mehrere Versuchsläufe nach dieser Vorschrift mit jeweils ca. 300 h Dauer ohne Unterbrechung der Wasser-und Kohlenwasserstoffzufuhr gefahren, ohen daß die Katalysatoraktivität nennenswert abklang. Vor jedem dieser Versuchsläufe wurde jeweils frischer Katalysator eingesetzt. Bei den übrigen Substraten (Versuche 2 bis 4) wurde im Unterschied hierzu eine Betriebsweise mit intermittierender Einspeisung des Kohlenwasserstoff-Einsatzgemisches gewählt, bei der nach jeweils 6-stündiger Dauer der Kohlenwasserstoff-Zufuhr der Kohlenwasserstoff-Strom abgeschaltet wird, während die Wasser-Zufuhr ununterbrochen weiterläuft.

Nachdem unter Anhebung der Temperatur in Reaktormitte auf ca. 650°C (= 923 K) 6 h lang nur Wasser eingespeist worden ist beginnt die Kohlenwasserstoff-Zufuhr von neuem, wobei im Katalysatorbett wieder eine Maximaltemperatur von ca. 630°C (= 903 K) eingestellt wird. Die Versuche 2 bis 4 wurden mit dieser Betriebsweise jeweils über ca. 100 h ausgedehnt, ohne daß ein merklicher Abfall der Katalysatoraktivität beobachtet wurde.

Die Aufarbeitung erfolgte bei allen Versuchen durch fraktionierende Destillation im Vakuum, wobei die in der Tabelle 2 angegebenen Fraktionen erhalten wurden, die die angestrebten Di-bzw. Trivinylbenzole enthielten.

Die Ergebnisse der Dehydrierversuche sind in der Tabelle zusammengestellt.

Tabelle 1: Ergebnisse der Dehydrierversuche

| Versuch Nr. | Hauptbestandteil des zur Dehydrierung eingesetzten aromatischen Kohlenwasserstoffgemisches: Formel | Gewichtsanteil % | Zusammensetzung des flüssigen Kohlenwasserstoffgemisches nach der Dehydrierung (1): Formel | (1) Gewichtsanteil % | (2): Formel | (2) Gewichtsanteil % |
|---|---|---|---|---|---|---|
| a 1 | 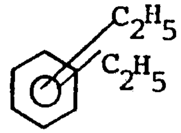 | 85 (0:m:p=0,2:3:1) | 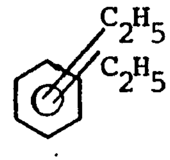 | 21,0 | 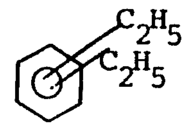 | 18,5 |
| a 2 | 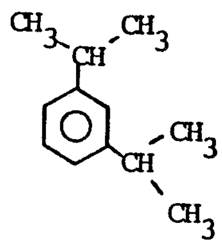 | 98,2 | 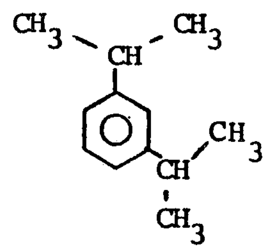 | 15,0 | 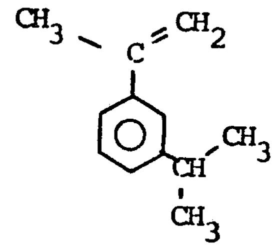 | 20,5 |
| a 3 | 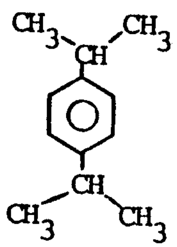 | 97,6 | 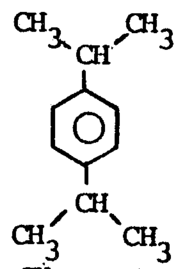 | 17,7 | 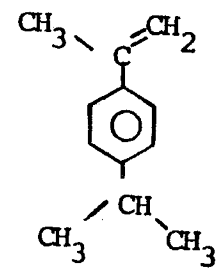 | 18,0 |
| a 4 | 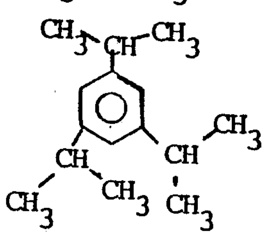 | 96,0 | 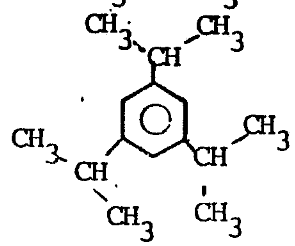 | 20,5 | 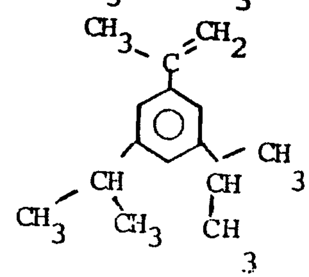 | 57,1 |

Tabelle 1 (Fortsetzung)

Zusammensetzung des flüssigen Kohlenwasserstoffgemisches nach der Dehydrierung

| Versuch Nr. | Zusammensetzung des flüssigen Kohlenwasserstoffgemisches nach der Dehydrierung (3) Formel | Gewichts-anteil % | (4) Formel | Gewichts-anteil % | (5) Formel | Gewichts-anteil % |
|---|---|---|---|---|---|---|
| a 1 | 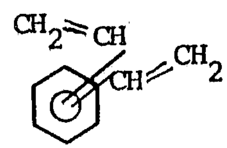 | 38,0 | - | - | nicht identifizierte Verbindungen | 22,5 |
| a 2 | 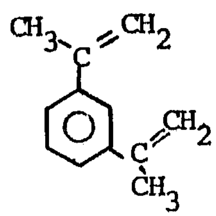 | 56,1 | - | - | nicht identifizierte Verbindungen | 8,4 |
| a 3 | 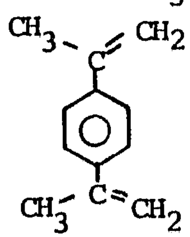 | 58,4 | - | - | nicht identifizierte Verbindungen | 6,0 |
| a 4 | 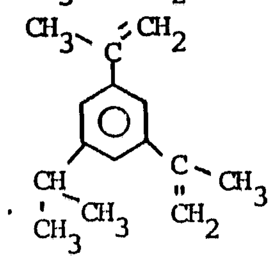 | 9,4 | - | 3,0 | nicht identifizierte Verbindungen | 10,0 |

<u>Tabelle 2</u> Ergebnisse der Aufarbeitung der Reaktionsgemische der Versuche 1-4 durch fraktionierende Vakuumdestillation

| Verbindung Nr. | Hauptbestandteil der zur weiteren Umsetzung verwendete Fraktion Formel | Gewichtsanteil % | Gewinnung der Fraktion aus Versuch Nr. |
|---|---|---|---|
| b | 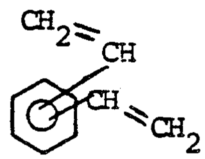 (o:m:p=0:3:1) | 80 | 1 |
| c | 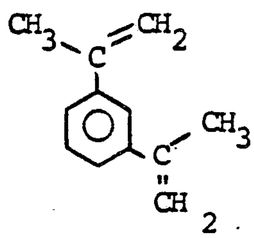 | 80 | 2 |
| d | 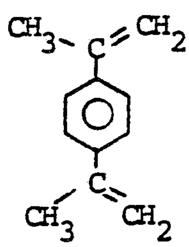 | 85 | 3 |
| e | 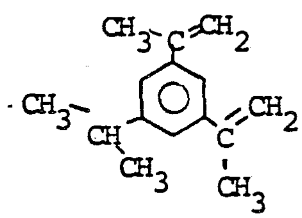 | 78 | 4 |
| f | 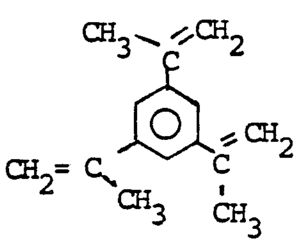 | 78 | 4 |

**Ansprüche**

1. Isoxazolidine der Formel I

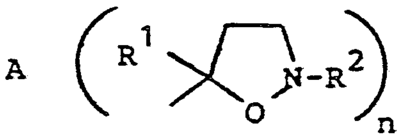

I

in welcher

n für 2 oder 3 steht,

R' für H oder Alkyl steht,

R² für gegebenenfalls substituiertes Alkyl, Cycloalkyl, Alkenyl, Alkinyl, Aryl sowie für gesättigte oder ungesättigte heterocyclische Reste die gegebenenfalls substituiert sind, steht,

A für zwei-oder dreiwertige Aryl-oder Heteroarylreste steht, die gegebenenfalls durch Substituenten aus der Reihe Alkyl, Cycloalkyl, Aryl, Aralkyl, Alkoxy, Alkylthio, Aryloxy, Arylthio, Halogen, CN, OH, Halogenalkyl, Halogenalkoxy, Halogenalkylthio, Alkoxyalkyl, gegebenenfalls halogensubstituiertes Methylendioxy oder Ethylendioxy substituiert sind.

Die neue Isoxazolidine der Formel I können in Form ihrer Enantiomere, Diastereomere sowie als Salze mit anorganischen oder organischen Säuren vorliegen.

2. Verfahren zur Herstellung der neuen Isoxazolidine der Formel I

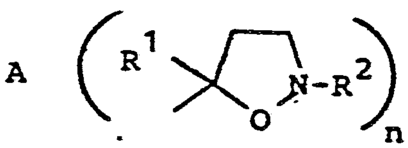

I

in welcher

n für 2 oder 3 steht,

R' für H oder Alkyl steht,

R² für gegebenenfalls substituiertes Alkyl, Cycloalkyl, Alkenyl, Alkinyl, Aryl sowie für gesättigte oder ungesättigte heterocyclische Reste die gegebenenfalls substituiert sind, steht,

A für zwei-oder dreiwertige Aryl-oder Heteroarylreste steht, die gegebenenfalls durch weitere Substituenten aus der Reihe Alkyl, Cycloalkyl, Aryl, Aralkyl, Alkoxy, Alkylthio, Aryloxy, Arylthio, Halogen, CN, OH, Halogenalkyl, Halogenalkoxy, Halogenalkylthio, Alkoxyalkyl, gegebenenfalls halogensubstituiertes Methylendioxy oder Ethylendioxy substituiert sind,

dadurch gekennzeichnet, daß man Hydroxylamine der Formel II

R²-NH-OH II

in welcher

R² die oben angegebene Bedeutung hat

mit Formaldehyd und Alkenen der Formel III

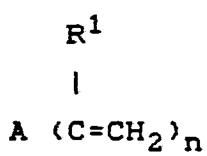

III

in welcher

A, R', n die oben angegebene Bedeutung haben

umsetzt.

3. Alkylsubstituierte Cycloalkylhydroxylamine der Formel IV

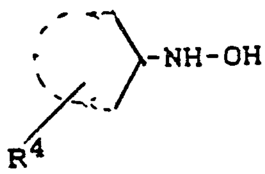

IV

in welcher

$R^4$ für Alkyl steht.

4. Verfahren zur Herstellung der alkylsubstituierten Cycloalkylhydroxylamine

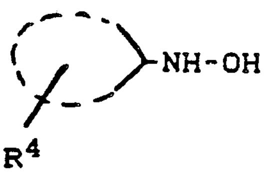

dadurch gekennzeichnet, daß man alkylsubstituierte Cycloalkanonoxime der Formel V

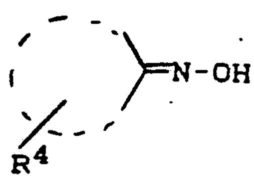

V

in welcher

R für Alkyl steht,

reduziert.

5. Schädlingsbekämpfungsmittel, gekennzeichnet durch einen Gehalt an mindestens einem Isoxazolidin der Formel I gemäß Anspruch 1.

6. Verwendung von Isoxazolidinen der Formel (I) gemäß Anspruch 1 zur Bekämpfung von Schädlingen.

7. Verfahren zur Bekämpfung von Schädlingen, dadurch gekennzeichnet, daß man Isoxazolidine der Formel (I) gemäß Anspruch 1 auf Schädlinge und/oder ihren Lebensraum einwirken läßt.

8. Verfahren zur Herstellung von Schädlingsbekämpfungsmitteln, dadurch gekennzeichnet, daß man Isoxazolidine der Formel (I) gemäß Anspruch 1 mit Streckmitteln und/oder oberflächenaktiven Mitteln vermischt.

9. 3,5-Diisopropenylcumol